Europäisches Patentamt

**European Patent Office**

Office européen des brevets

(11) Publication number: **0 212 837**
A2

# (12) EUROPEAN PATENT APPLICATION

(21) Application number: **86305400.3**

(22) Date of filing: **14.07.86**

(51) Int. Cl.⁴: **A 61 L 9/01**, A 61 L 9/04

(30) Priority: **12.07.85 JP 153738/85**

(43) Date of publication of application: **04.03.87 Bulletin 87/10**

(84) Designated Contracting States: **DE FR GB IT SE**

(71) Applicant: **CLEANLITE AIRE INC., 74 Gervais Drive, Don Mills Ontario, M3C 1Z3 (CA)**

(72) Inventor: **Suzuki, Atsushi, 3-2-402, Kasumigaoka 1-chome, Kamifukuoka-shi Saitama-ken (JP)**

(74) Representative: **Johnson, Terence Leslie, Edward Evans & Co. Chancery House 53-64 Chancery Lane, London WC2A 1SD (GB)**

(54) **Deodorizing agent.**

(57) Aqueous compositions of glyoxal containing calcium chloride and an organic humectant such as ethylene glycol have a longer term deodorizing capability than do comparable compositions in the absence of the humectant.

EP 0 212 837 A2

- 1 -                                    P 1671-1 EPO

## DEODORIZING AGENT

The present invention relates to glyoxal based deodorizing compositions in removing offensive odors such as are associated with activated nitrogen compounds and activated sulfur compounds and the like.

Glyoxal has the nature such that it chemically reacts with activated nitrogen compounds such as ammonia and amines and activated sulfur compounds such as hydrogen sulfide or mercaptans and various other kinds of materials having an offensive odor, and changes them to odorless compounds. Due to their nature, glyoxal based deodorizing compositions are widely used as a deodorizing agents in the industrial field and in home use.

Glyoxal based deodorizing compositions normally comprise aqueous solutions of glyoxal. It is known that when in contact with water, glyoxal exists in polymeric forms. It will be appreciated that since it is desirable for effective deodorizing action that there be a relatively large surface area of the glyoxal based deodorizing composition exposed to the atmosphere, the compositions are often impregnated onto materials such as felt, spunge, paper or porous substrates, from which the water component of the composition evaporates relatively rapidly. Even where the compositions are not impregnated onto substrates, evaporation of the water component takes place. At about 80% concentration of the glyoxal, this then becomes a gel, and the deodorizing effectiveness of such gel is relatively low. Thus, conventional glyoxal deodorizing compositions are inadequate for applications where the deodorizing effect must be maintained for a long period of time, such as in refrigerators, toilets, patient's rooms, locker rooms, dressing rooms, and the like.

To solve this problem, there has been tried a method whereby the evaporation of the water component is reduced by mixing aqueous solutions with calcium chloride. However, the duration period of the deodorizing effect is still insufficient.

It is an object of the present invention to provide glyoxal based deodorizing compositions having an effective deodorizing capability for increased periods of time.

It is a further object of the invention to provide substrates impregnated with the improved deodorizing compositions.

In accordance with a first aspect of the invention, the glyoxal based deodorizing compositions comprise aqueous solvents containing an organic humectant. Such humectants typically comprise water soluble lower glycols and polyols having a relatively low vapour pressure under normal ambient conditions. They are hydrophilic in nature and act to retain the water in the system, thereby reducing formation of the polyglyoxal gel, and so increasing the time-effectiveness of the deodorizing compositions and substrates inpregnated therewith.

Preferably, the compositions include therein calcium chloride, which also serves to reduce the evaporation of water from the system.

Practically speaking, in this invention, the blending amounts of the principal components of the composition are respectively determined in accordance with the purpose of the deodorization. Namely, assuming that the component weight of glyoxal of the deodorizing fluid of the glyoxal system is 100 parts, the component weight of calcium chloride is set to a value within a range of 25 to 100 parts, and the component weight of an organic humectant is set to a value within a range of 20 to 100 parts.

In other words, the blending amounts are changed in accordance with the humidity and temperature at the location where the deodorizing agent is used. In general, it is preferable to increase an amount of calcium chloride in the condition of high temperature or low humidity, while it is desirable to decrease an amount of calcium chloride in the condition of low temperature or high humidity.

In this invention, calcium chloride is mixed as a moisture absorbent into the glyoxal deodorizing fluid. Thus, the evaporation of the water in the glyoxal deodorizing fluid is suppressed and an increase in glyoxal concentration due to the evaporation of the water is suppressed.

In addition, according to the invention, the organic humectant is further mixed to the glyoxal deodorizing fluid blended with calcium chloride. Thus, the deodorizing capability of glyoxal is effectively held for a long period of time in cooperation with the function of calcium chloride. This is because it is presumed that organic humectant functions as a kind of surface active agent and the moving force of glyoxal in the deodorizing fluid increases, so that unreacted glyoxal is always actively supplied to the reaction surface of the material having the offensive oder.

Organic humectants comprise, for example, ethylene glycol and propylene glycol, and polyglycols, glycerol, water soluble polyvinyl alcohol (pva) and water solube cellulose materials, and the suitability of these and other humectants as known in the art will be gauged from their effectiveness in reducing the evaporation of water from the system, their toxicologic properties in relation to any particular use to which the deodorizing composition may be put, and also from the economic standpoint, and it is possible that humectants other than those specifically mentioned may be preferred according to particular circumstances. It is also apparent that mixtures comprising more than one humectant may be utilized.

The effects of the invention will then be summarized hereinbelow in relation to the preferred embodiment. In this embodiment calcium chloride and ethylene glycol are blended to the glyoxal deodorizing fluid, so that the evaporatin of the water in the glyoxal deodorizing fluid is suppressed and also glyoxal is activated. Therefore, according to the invention, the deodorizing capability of glyoxal can be effectively utilized for a long period of time. As will be apparent from the preferred embodiments and comparison examples, which will be mentioned hereinafter, according to the embodiments of the invention, the duration of the deodorizing effect is increased several fold as compared with the conventional one. Consequently, the deodorizing agent of the invention can be used for such an application that the deodorizing effect must be held for a long period of time, such as in the refrigerators, automobiles, warehouses, toilets, patient's rooms, locker rooms, dressing rooms, and the like.

Embodiments of the present invention and comparison examples will then be shown hereinbelow.

When it is assumed that the component weight of glyoxal of the glyoxal system deodorizing fluid is 100 parts, the blending amounts of calcium chloride and ethylene glycol are determined in accordance with the use object of the deodorizing fluid in a manner such that the component weight of calcium

0212837
P 1671-1 EPO

chloride lies within a range of 25 to 100 parts and the component weight of ethylene glycol lies within a range of 20 to 100 parts, respectively.

For example, as shown in Table 1, four samples A to D are prepared. Namely, sample A is the deodorizing fluid as the embodiment and consists of glyoxal of 40g, calcium chloride of 30g, ethylene glycol of 20g, and water of 90g. Samples B to D are comparison examples. Sample B is the deodorizing fluid which is the same as sample A except that ethylene glycol is not contained in sample B. Sample C is the deodorizing fluid consisting of glyoxal of 40g and water of 120g and containing no calcium chloride and no ethylene glycol. Sample D is the pure water. Four cubes of 4 cm of sponge of the cellulose system were prepared and impregnated with sample A to D by an amount of 35g, respectively. These cubes were left in the refrigerator and the time-elaspe changes in weight were measured to obtain the amounts of water decreased. The results of the measurement are shown in Table 2. Numerals added to sample numbers A to D denote the different samples as the deoderizing fluids having the smae mixture ratio.

## TABLE 1

| SAMPLE | BLENDING AMOUNT (g) | | | |
|---|---|---|---|---|
| | GLYOXAL | CALCIUM CHLORIDE | ETHYLENE GLYCOL | WATER |
| A | 40 | 30 | 20 | 90 |
| B | 40 | 30 | 0 | 90 |
| C | 40 | 0 | 0 | 120 |
| D | 0 | 0 | 0 | 100 |

## TABLE 2

| SAMPLE | RESULTS OF MEASUREMENT OF WEIGHT DECREASE AMOUNTS (g) | | | | | | |
|---|---|---|---|---|---|---|---|
| | 1 day | 3 days | 10 days | 1 month | 2 months | 3 months | 6 months |
| A1 | 1.6 | 3.8 | 8.0 | 11.5 | 11.8 | 12.0 | 12.0 |
| A2 | 1.8 | 4.0 | 8.5 | 12.0 | 12.0 | 12.5 | 12.2 |
| B1 | 2.0 | 4.5 | 10.0 | 12.2 | 13.0 | 13.2 | 13.0 |
| B2 | 1.7 | 4.5 | 10.2 | 12.5 | 13.0 | 13.2 | 13.5 |
| C1 | 2.5 | 6.0 | 21.0 | 27.5 | 28.0 | 28.0 | 28.0 |
| C2 | 2.7 | 6.7 | 21.7 | 28.0 | 28.0 | 28.0 | 28.0 |
| D1 | 3.2 | 7.7 | 28.7 | 33.5 | 34.5 | 34.5 | 34.5 |
| D2 | 3.2 | 7.7 | 28.7 | 34.0 | 34.5 | 34.5 | 34.5 |

The surfaces of the sponges impregnated with the deodorizing fluid A and B are wet even after six months have elapsed. The surfaces of the sponges impregnated with the deodorizing fluid C and water D are dry after a expiration of six months. Thus, the deodorizing fluid A according to the invention exhibited the excellent result in terms of the suppression of the evaporation of the water.

On the other hand, the filters consisting of fifty-five paper tubes each having the dimensions of (10 x 15 x 1 mm) were impregnated wit the deodorizing fluids A, B and C by an amount of 20g, respectively. The filters impegnaed with these deodorizing fluids were enclosed in the casing havin a motor and a fan (the blowing capability of the fan is 0.75 /min). The gas containing ammonia of 50 ppm and hydrogen sulfide of 20 ppm as concentrations was poured from the suction inlet into the casing and circulated therein. The concentrations of residual ammonia and hydrogen sulfide were detected by the Kitagawa's detector tube at the location 10cm away from the exhaust port. The time-elapse changes of the concentrations were measured and the results are shown in Table 3.

TABLE 3

| SAM-PLE | CONCEN-TRATION AT SUC-TION PORT (ppm) | RESULTS OF MEASUREMENTS OF RESIDUAL CONCENTRATIONS AT THE EXHAUST PORT (ppm) | | | | | |
|---|---|---|---|---|---|---|---|
| | | 3 day | 10 days | 1 month | 2 months | 3 months | 6 months |
| A | AMMONIA | 5 | 5 | 5 | 5 | 8 | 10 |
| B | 50 ppm | 5 | 5 | 15 | 45 | 50 | 50 |
| C | | 20 | 45 | .50 | 50 | 50 | 50 |
| A | HYDROGEN | 2 | 2 | 2 | 2 | 2 | 2 |
| B | SULFIDE | 2 | 2 | 4 | 15 | 20 | 20 |
| C | 20 ppm | 10 | 15 | 20 | 20 | 20 | 20 |

The filter impregnated with the deodorizing fluid A still maintains the deodorizing capability even after six months have elapsed. On the other hand, the filters impregnated with the deodorizing fluids B and C completely lost the deodorizing capability. Thus, the deodorizing fluid A of the invention exhibited the excellent result in terms of the duration of the deodorizing effect.

The present invention is not limited to the foregoing embodiments but many variations are possible within the spirit and scope of the appended clam of the invention.

CLAIMS:

1.      A deodorizing composition comprising glyoxal and an organic humectant.

2.      A deodorizing composition comprising glyoxal and an organic humectant, together with calcium chloride.

3.      Deodorizing compositions as defined in Claim 2, wherein said glyoxal comprises 100 parts by weight, said organic humectant comprises from 25 to 100 parts by weight, and said organic humectant comprises from 20 to 100 parts by weight.

4.      Deodorizing compositions as defined in Claim 1, 2 or 3, wherein said organic humectant is selected from the group consisting of water soluble lower glycols and polyols.

5.      Deodorizing compositions as defined in Claim 1, 2 or 3, wherein said organic humectant is selected from the group consisting of ethylene glycol, propylene glycol, glycerol, water soluble polyvinyl alcohol and water soluble polyglycols and water soluble cellulose material.

6.      Deodorizing compositions as defined in Claim 1, 2 or 3, wherein said organic humectant is selected from ethylene glycol and propylene glycol.

7.      A substrate impregnated with an aqueous solution of the deodorizing composition of Claim 1, 2 or 3.

8.      A substrate impregnated with an aqueous solution of the composition of Claim 1, 2 or 3, wherein said organic humectant is selected from the group consisting of water soluble lower glycols and polyols.

9.      A substrate impregnated with an aqueous solution of the composition of Claim 1, 2 or 3, wherein said organic humectant is selected from

0212837

P 1671-1 EPO

the group consisting of ethylene glycol, propylene glycol, glycerol, water soluble polyvinyl alcohol and water soluble polyglycols and water soluble cellulose material.

10. A substrate impregnated with an aqueous solution of the composition of Claim 1, 2 or 3, wherein said organic humectant is selected from ethylene glycol and propylene glycol.